# EUROPÄISCHE PATENTSCHRIFT

(11) **EP 0 302 127 B1**
(45) Veröffentlichungstag und Bekanntmachung des Hinweises auf die Patenterteilung: **07.04.1993**
(21) Anmeldenummer: 87111196.9
(22) Anmeldetag: 03.08.1987
(51) Int. Cl.: A61B 5/00, G01N 27/416

(54) **Polarographisch-amperometrischer Messwertaufnehmer**
Polarographic-amperometric sensor
Détecteur polarographique et ampèremétrique

(43) Veröffentlichungstag der Anmeldung: 08.02.1989
(73) Patentinhaber: PPG Hellige GmbH, 79007 Freiburg (DE)
(72) Erfinder: Ahsbahs, Walter, D-7802 Merzhausen (DE); Leist, Helmut, D-7808 Waldkirch (DE)
(74) Vertreter: TER MEER STEINMEISTER & PARTNER GbR

(56) Entgegenhaltungen:
- EP-A- 0 102 033
- DE-A- 3 408 800
- IEEE TRANSACTIONS ON BIOMEDICAL ENGINEERING, Band BME-31, Nr. 12, Dezember 1984, Seiten 792-800, New York, US; Y. MENDELSON et al.: "Noninvasive Transcutaneous Monitoring of Arterial Blood Gases"
- THE LANCET, 24. Mai 1980, Seiten 1111-1113, London, GB; M.D. WHITEHEAD et al.: "Transcutaneous estimation of arterial PO2 and PCO2 in newborn infants with a single electrochemical sensor"
- JOURNAL OF APPLIED PHYSIOLOGY, Band 51, 1981, Seiten 1027-1032; J.W. SEVERINGHAUS "A combined transcutaneous PO2-PCO2 electrode with electrochemical HCO3-Stabilization"

## Beschreibung

Die Erfindung betrifft einen Meßwertaufnehmer zur transcutanen Bestimmung des Sauerstoffpartialdrucks (tcpO₂) im Blut eines Lebewesens mittels einer polarographisch-amperometrischen Elektrodenanordnung nach CLARK oder einen anderen polarographisch-amperometrischen Meßwertaufnehmer mit CLARK-Anordnung, bei der an der Meßfläche freiliegend und mit einer durch eine dünne Membran überspannten Elektrolytschicht bedeckt wenigstens eine in ein Isoliermaterial eingebettete Kathode und wenigstens eine Edelmetallanode in einen Körper eingesetzt sind, der normalerweise zugleich als Referenz mittels Silber/Silberchlorid (Ag-AgCl) dient.

Transcutane Sauerstoffpartialdruckmessungen (tcpO₂-Messungen) werden bisher meist polarographisch-amperometrisch, beispielsweise mit einer Zwei-Elektrodenanordnung durchgeführt. Als besonderes Problem tritt dabei aber der Nachteil auf, daß sich eine Silber/Silberchlorid-Referenzschicht (Ag/AgCl-Referenz) durch anodisches Chlorieren bildet, da die Anode und die Referenz zu einer Elektrode aus Ag bei Verwendung eines Cl-haltigen Elektrolyten zusammengefaßt werden. Eine Elektrodenanordnung dieser bekannten Art ist jedoch hinsichtlich ihrer Langzeitbetriebsstabilität nicht zufriedenstellend. Es findet ein elektrolytischer Ag-Transport zur Kathode statt, wobei sich Ag an der Kathode ablagert und damit vor allem bei sogenannten Microkathoden deren Fläche und damit der pO₂-Meßstrom verändert werden. Gelegentlich bilden sich sogar rasch wachsende Ag-Dendriten, insbesondere wenn nicht sehr dünne Elektrolytschichtdicken von nur wenigen µm verwendet werden.

Bei gleichzeitiger transcutaner Messung des Kohlendioxidpartialdrucks (pCO₂) nach Stow-Gertz-Severinghaus im selben Elektrolyten mittels eines kombinierten Meßwertaufnehmers (tcpO₂/tcpCO₂-Meßwertaufnehmer) wird außerdem der pH-Wert durch die kathodische Bildung von OH-Ionen beeinflußt.

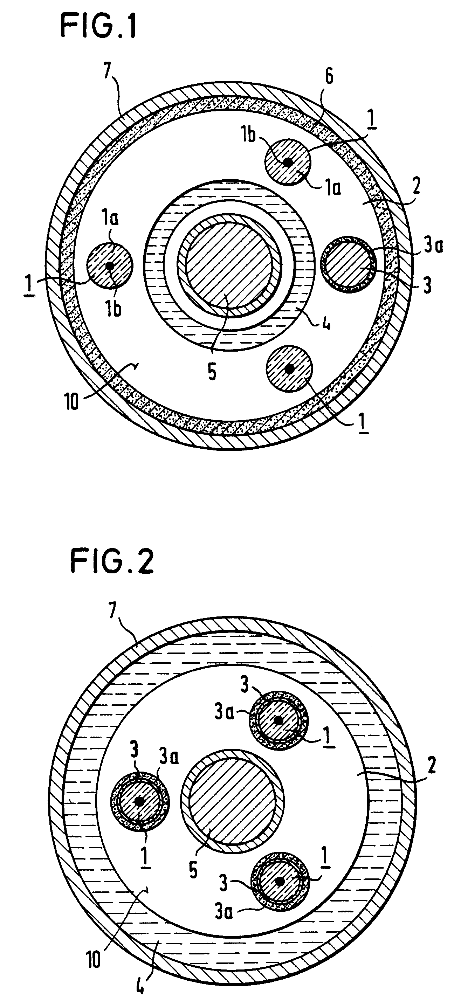

Um diese Effekte zu beheben, bietet sich eine bekannte Drei-Elektrodenanordnung zur polarographisch-amperometrischen pO₂-Messung an, wie sie beispielsweise für einen kombinierten Meßwertaufnehmer durch Severinghaus in "Journal of Applied Physiology", Vol. 51/1981, Seite 1027 ff (vgl. insbesondere Fig. 2 auf Seite 1028) beschrieben wurde, aber auch in anderen Ausführungsformen bekannt ist. Bei einer solchen Mehrfach-Elektrodenanordnung wird die Funktion von Anode und Referenz aufgeteilt, so daß insbesondere auch eine Freiheit in der Materialauswahl für die Anode besteht, so daß z. B. Platin (Pt) verwendet werden kann, oder auch Gold (Au) oder unter anderem auch das gleiche Material wie für die Kathode. Dadurch kann erreicht werden, daß an der Anode die gleiche Zahl von OH-Ionen verbraucht wird wie an der Kathode erzeugt wurden.

Bei der für transcutane Meßwertaufnehmer (Sensoren erforderlichen dünnen Elektrolytschicht zeigt sich jedoch, daß entgegen den üblichen Dimensionen die Referenz (der Referenzkörper) aus Stabilitätsgründen, insbesondere bei dem erwähnten kombinierten Sensor, relativ großflächig ausgebildet werden muß, die Gegenelektrode (Anode) dagegen eher eine kleine Fläche annehmen sollte. Hierbei entsteht jedoch wiederum eine gewisse Ag⁺-Ionenkonzentration im Elektrolyten, was wiederum zu Ag-Ablagerungen an der Kathode führt. Die Meßflächen solcher Sensoren müssen also von Zeit zu Zeit und insbesondere vor jedem neuen Meßvorgang poliert werden. Dies gilt auch für den oben erwähnten, von Severinghaus beschriebenen kombinierten Meßwertaufnehmer.

Der Erfindung liegt damit die Aufgabe zugrunde, tcpO₂-Meßwertaufnehmer und/oder die erwähnten kombinierten tcpO₂-/tcpCO₂-Meßwertaufnehmer so zu verbessern, daß die erläuterten störenden und insbesondere den pO₂-Meßstrom verändernden Ag-Ablagerungen an der Kathode einerseits bzw. unerwünschte Veränderungen der OH-Ionenkonzentration im Elektrolyten andererseits vermieden werden.

Die Erfindung ist bei einem transcutanen Meßwertaufnehmer gemäß Oberbegriff Anspruch 1 dadurch gekennzeichnet, daß die Gegenelektrode durch einen das Isoliermaterial (in der Regel Glas) der Arbeitselektrode wenigstens teilweise umschließenden Ring aus inertem, leitendem oder dotiertem, halbleitendem Elektroden material gebildet ist.

Die erfindungsgemäße Lehre geht also dahin, die Anode als eine die Kathode(n) wengistens im Bereich der Meßfläche umschließende gekrümmte Fläche(n) bzw. Ring(e) auszubilden, der (bzw. die) die Referenz geometrisch von der Kathode (bzw. den Kathoden) trennt (trennen). Die Anode, d. h. die Gegenelektrode, wird also nicht von der Kathode entfernt angeordnet, wie das beispielsweise bei dem Kombi-Sensor nach Fig. 2 der erwähnten Literaturstelle Severinghaus bewußt vorgesehen ist. Es wird auch nicht wie dort eine punktförmige Anordnung einer Anode innerhalb der Referenzfläche gewählt, wie dies auch bei anderen bekannten pO₂- oder pO₂/pCO₂-Meßwertaufnehmern der Fall ist.

Die vorteilhafte Wirkung dieser neuartigen Ausbildung der Anode beruht vor allem auf folgendem: Für die Anode kann ein Material gewählt werden, das ein ausreichend positiveres Potential als das der Referenz bei potentiostatischer Steuerung - wie üblich - aufweist. Dadurch entsteht für die Ag⁺-Ionen eine Potentialschwelle, die sie bei dünner Elektrolytschicht nicht überwinden können. Dadurch werden Ag-Ablagerungen an der Kathode vermieden. Ein Polieren der Meßfläche des Meßaufnehmers zur Entfernung von Ag-Ablagerungen ist also nicht mehr erforderlich. Dies ist für die Praxis des Messens mit solchen transcutanen Meßwertaufnehmern im klinischen Routinebetrieb ein großer Vorteil.

Wird die Anode vorteilhafterweise nur als schmale(r), die Kathode(n) umgebende(r) Ring(e) ausgeführt, so wird die Referenz nicht beeinträchtigt. Das Elektrolytpotential wird eindeutig durch das Referenzpotential bestimmt.

Die Anode kann z. B. als dünnes Edelmetallblech um das Isoliermaterial der Kathode(n) gewickelt oder aufgedampft bzw. aufgesputtert sein, wobei, wenn es sich um (eine) Microkathode(n) handelt, die Kathode(n) stets in ein Glasröhrchen eingeschmolzen sind.

Der Abstand zwischen Anode und Kathode(n) wird in der Regel dabei so klein, daß auch ein nicht ausgeregelter Spannungsabfall an der Kathode bei kleinem pO₂-Meßstrom nicht ins Gewicht fällt.

Als weiterer wesentlicher Vorteil der Erfindung sei auf die Wirkung der O₂-Erzeugung an der Anode in geringsmöglicher Entfernung vom O₂-Verbrauch an der Kathode hingewiesen. Hierdurch läßt sich der Nachteil der O₂-verbrauchenden Messung, verursacht durch einen O₂-Gradienten, auch innerhalb der Haut und damit eine nicht im vorhinein kalibrierbare Meßwertabweichung mindern bzw. ganz beseitigen.

Als weitere vorteilhafte Wirkung der Erfindung hat sich überraschenderweise gezeigt, daß eine Ag-Ablagerung an der Kathode und als Folge davon eine Drift des pO₂-Meßstroms oder eines sonstigen polarographisch-amperometrischen Meßstroms (beispielsweise bei gleichzeitiger tcpCO₂-Messung) auch dann nicht auftritt, wenn die die Anode bildenden Edelmetallringe um die Kathode nicht durch einen äußeren Kreis beschaltet sind, so daß der Strom letztlich zur Referenz fließt.

Hierbei wird der Meßstrom jedoch von der Referenz her auf dem Weg über den (die) Edelmetallring(e) der Anode fließen, wobei dieser (diese) mit der Referenz als z. B. Pt-Ag/AgCl-Zelle oder Au-Ag/AgCl-Zelle mit einer EMK (gemessen wurden Werte von -40 mV bis -60 mV bzw. +10 mV bis + 30 mV) und einem Innenwiderstand betrachtet werden kann (können). Diese Zelle beeinträchtigt die amperometrische Messung nicht, aber der (die) Edelmetallring(e) gibt (geben) zunächst seinerseits (ihrerseits) keine Ag-Ionen an die Kathode weiter. Diese lagern sich bestenfalls auf ihm (ihnen) ab.

Um nun zu gewährleisten, daß auch bei längerer Betriebsdauer keine Beschichtung des Edelmetallrings (der Edelmetallringe) mit Silber stattfindet und damit wieder Ag-Ionen zur Kathode gelangen können, wird als weitere vorteilhafte Ergänzungsmaßnahme vorgeschlagen, eine äußere (oder auch integrierte innere) elektrische Verbindung zwischen dem (den) Edelmetallring(en) der Anode und der Ag/AgCl-Referenz vorzusehen. Messungen haben ergeben, daß diese elektrische (galvanische) Verbindung ohne nachteilige Einflüsse ist, sofern der (die) anodenseitige(n) Edelmetallring(e) in Relation zur Fläche der Referenz kleinflächig ausgebildet ist (werden), was in der Regel immer der Fall sein wird, insbesondere wenn, wie heute üblich, (eine) Microkathode(n) verwendet wird (werden). Dadurch wird erreicht, daß der Kathodenstrom aufgrund der Konzentration des elektrischen Feldes auf die nächstliegende Metallkante, sofern die erwähnte dünne Elektrolytschicht im Bereich des (der) die Anode bildenden Edelmetallrings (Edelmetallringe) vorliegt, zu diesem (diesen) hinfließen und nicht zur oder entlang der Ag/AgCl-Referenz, so daß ein aktiver Ag-Transport vermieden wird.

Die erwünschte dünne Elektrolytschicht im Bereich des (der) die Anode bildenden Edelmetallrings (Edelmetallringe) kann auch dann gewährleistet werden, wenn in der Meßfläche des Meßaufnehmers ein sogenanntes Elektrolytreservoir vorgesehen ist, das beispielsweise bei der oben erwähnten kombinierten pO₂/pCO₂-Meßanordnung von Severinghaus als halbringförmige Nut ausgebildet ist. Bei einer vorteilhaften Ausführungsform der Erfindung wird dieses Elektrolytreservoir in radialer Erstreckung zwischen den von den Anodenringen umgebenen Kathoden angeordnet, wenn, wie bei manchen Meßwertaufnehmern dieser Art vorgesehen, mehrere, beispielsweise drei, Microkathoden verwendet werden.

Die Erfindung und vorteilhafte Einzelheiten werden nachfolgend anhand von Ausführungsbeispielen durch die Zeichnungen weiter veranschaulicht. Es zeigen:
- Fig. 1: zur vergleichenden Veranschaulichung eine Draufsicht auf die Meßfläche eines dem Stand der Technik entsprechenden transcutanen pO₂/pCO₂-Meßwertaufnehmers,
- Fig. 2: ein erstes Ausführungsbeispiel eines Meßwertaufnehmers mit erfindungsgemäßen Merkmalen, ebenfalls dargestellt als kombinierter pO₂/pCO₂-Sensor,
- Fig. 3: eine abgewandelte Ausführungsform eines Meßwertaufnehmers mit erfindungsgemäßen Merkmalen mit einer vorteilhaften Anordnung von mehreren Elektrolytreservoirs in der Meßfläche,
- Fig. 4: eine vorteilhafte Abwandlung der in Fig. 3 dargestellten Anordnung; und
- Fig. 5: eine weitere Gestaltung der Meßfläche eines Meßwertaufnehmers mit erfindungsgemäßen Merkmalen, wobei anstelle von drei Kathoden zwei Paare von Kathoden sowie eine Elektrolytrinne entsprechend dem Konzept von Fig. 4 vorgesehen sind.

Die etwa im Maßstab 10:1 vergrößerte schematische Draufsichtdarstellung auf die Meßfläche eines bekannten, im Prinzip der Konstruktion von Severinghaus nach Fig. 2 der oben genannten Literaturstelle entsprechenden kombinierten pO₂/pCO₂-Meßwertaufnehmers, verdeutlicht als räumlich größtes Teil der Elektrodenanordnung eine Ag/AgCl-Referenz 2 (Referenzkörper), die in bekannter Weise auch durch eine andere geeignete Festkörperreferenz ersetzt sein kann. Eine umgebende Isolationsschicht 6 wird vom Rand 7 der Meßfläche umschlossen, an dem die Meßmembran in bekannter Weise nach CLARK aufgespannt wird. Der Meßaufnehmer nach Fig. 1 enthält beispielsweise drei den Referenzkörper senkrecht zur Zeichenebene durchsetzende Microkathoden 1, die beispielsweise durch einen in ein Glasröhrchen 1a eingeschmolzenen Platindraht 1b gebildet sind, der an der dargestellten polierten Meßfläche freiliegt und am gegenüberliegenden, von der Meßfläche entfernten Ende in bekannter Weise über eine Ader eines Leitungsbündels an eine Meßschaltung angeschlossen ist, deren erster Teil (z. B. Vorverstärkerstufe) auch bereits in das Gehäuse des Meßwertaufnehmers integriert sein kann. Die Gegenelektrode oder Anode z. B. aus Platin oder Gold ist mit Bezugshinweis 3 angegeben und wird von einem dünnen isolierenden Mantel 3a umgeben. Der isolierte Draht der Anode 3 liegt in der Ebene der Meßfläche ebenfalls plan poliert frei, wie die Flächen der Kathoden 1; er ist auf der der Meßfläche entfernten Seite im Gehäuse des Meßaufnehmers ebenfalls an eine Ader eines Anschlußkabels bzw. an eine vorverarbeitende integrierte Schaltung angeschlossen. In der Achse, also in Zentralposition des Meßaufnehmers befindet sich eine bekannte pH-Elektrode 5, die als Glaselektrode oder mit besonderen Vorteilen als Iridium/Iridiumoxid-Elektrode gemäß EP-A-0 102 033 ausgebildet sein kann. Im dargestellten Ausführungsbeispiel ist die zur CO₂-Konzentrationsbestimmung vorgesehene pH-Elektrode 5 durch eine Ringnut 4 umgeben, die als Elektrolytreservoir dient. Der Zweck und besondere Vorteil eines solchen in der Meßfläche liegenden Elektrolytreservoirs ist in der Fachwelt bekannt und beispielsweise auch in der oben erwähnten Literaturstelle Severinghaus erläutert. Bei der von Severinghaus beschriebenen Konstruktion eines kombinierten Meßwertaufnehmers allerdings wird das Elektrolytreservoir durch ein kreisförmiges Nutsegment gebildet, wobei die Anode unmittelbar in einem dieser Nutsegmente liegt.

Im Gegensatz zur bekannten Ausführungsform nach Fig. 1 wird beim ersten Ausführungsbeispiel der Erfindung gemäß Fig. 2 die Anode 3 durch eine dünne, das Isoliermaterial (Glas) der Microkathoden 1 umgebende Edelmetallbeschichtung in Form eines Rings gebildet, der gegen die Referenz 2 wiederum durch eine Isolierschicht 3a galvanisch getrennt ist. Es sind also im dargestellten Beispiel drei Teilanoden entsprechend der Anzahl der Microkathoden 1 vorhanden. In anderen Worten, die Kathode(n) ist (sind) durch die ringförmig umschließende (Teil-)Anode 3 geometrisch von der Referenz 2 getrennt. Die Ringe der (Teil-)Anoden können, wie erwähnt, durch ein dünnes Edelmetallblech oder vorteilhafterweise durch Aufdampfen oder Aufsputtern sowie in Schichttechnik gebildet sein. Die Ringe 3 der Anode sind gegebenenfalls zum der Meßfläche entfernten Ende des Meßaufnehmers elektrisch herausgeführt, beispielsweise dadurch, daß die Ringe 3 sich als hülsenartige Ummantelung der Kathoden in axialer Richtung senkrecht zur Zeichenebene fortsetzen. Die nutartige Vertiefung 4 als Elektrolytreservoir liegt in radial weiter außen stehender Position. Hierdurch ist ein größeres Elektrolytreservoir möglich gegenüber Fig. 1 und eine größere Heizfläche in Kathodennähe im Vergleich zur Ausführungsform nach Fig. 2.

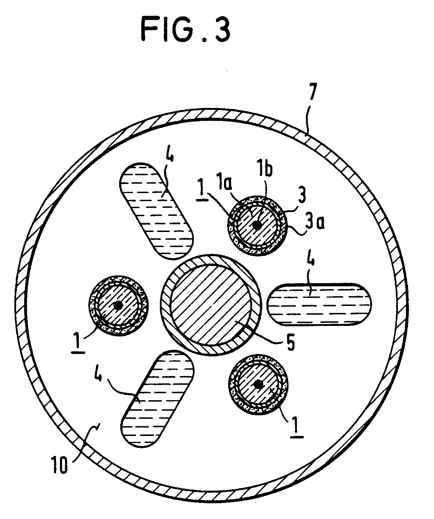

Bei der Ausführungsform nach Fig. 3 ist die Meßfläche eines ebenfalls kombinierten pO₂/pCO₂-Meßaufnehmers etwas anders gestaltet als bei der Ausführungsform nach Fig. 2. Hier liegen drei Radialnuten oder Radialrinnen 4 als Elektrolytreservoir jeweils in einer den Winkelabstand zwischen der Kathoden/Anoden-Kombination 1, 3 unterteilenden Position. Hierdurch wird bei ausreichendem Elektrolytvolumen eine gute Elektrolytversorgung aller Elektroden sichergestellt.

Des weiteren zeigt es sich, daß die Ablagerung von Silber an der (auf den) Kathode(n) auch dadurch wesentlich reduziert wird, daß die Meßfläche selbst keine Ag-AgCl-Referenz enthält, sondern daß das Silber in der Fläche blank bleibt oder gar isoliert wird und daß nur eine bzw. einzelne Referenzstellen in Form von Ag-AgCl ausgebildet bzw. das Silber nur an diesen Stellen chloriert wird. Vorzugsweise bieten sich hierzu die mechanisch geschützten Elektrolytrinnen 4 an.

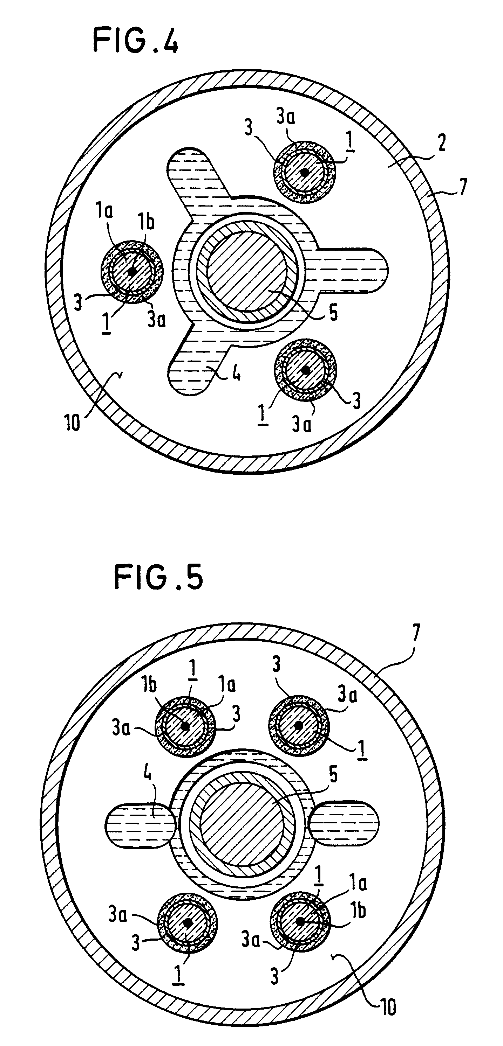

Die in Fig. 4 gezeigte Anordnung verknüft die in Fig. 1 und Fig. 3 gezeigte Anordnung der Elektrolytrinne 4 in der Weise, daß die Elektrolytversorgung der mittigen pH-Elektrode 5 im Vergleich zu Fig. 3 noch weiter verbessert wird bei gleichzeitiger Wahrung des verfügbaren Elektrolytvolumens und der Heizfläche in Kathodennähe.

Die in Fig. 5 gezeigte Anordnung zeigt eine Variante der Anordnung von Fig. 4 mit vier statt drei Kathoden und den erfindungsgemäß angebrachten Anodenringen 3.

Eine besonders vorteilhafte Ausführung ergibt sich, wenn die Ag/AgCl-Referenz ganz oder überwiegend nur in den Elektrolytrinnen 4 ausgebildet wird, wodurch weniger AgCl in Lösung geht bzw. besser in Kathodennähe zur Verfügung steht.

Bei den radialen Elektrolytrinnen nach den Ausführungsformen der Fig. 3, 4 und 5 wird eine gleichmäßige Verteilung des Elektrolytreservoirs in der Nähe aller Elektroden erreicht.

Die erfindungsgemäß vorgesehene Elektrodenanordnung ist auch für Messungen mit Kathetern von Bedeutung, wobei kein wärmeleitendes Material für thermostatische Zwecke vorgesehen wird. Die Methode kann auch allgemein für polarographischamperometrische Meßanordnungen nach CLARK angewandt werden, d. h. auch bei der kathodischen Reduktion anderer Stoffe, z. B. N₂O, oder bei der anodischen Oxidation, z. B. von Alkoholen, wobei ebenfalls die Messung störende Ablagerungen vermieden oder zumindest vermindert werden können, indem in diesem Fall zwischen Arbeitselektrode (bei pO₂ die Kathode) und Referenz (im obigen Fall Ag-AgCl) ein Gegenelektrodenring (bei pO₂ die Anode) erfindungsgemäß eingefügt wird. Dieser kann auch anstelle von Edelmetall, wie Platin oder Gold, aus anderen inerten leitfähigen Materialien gebildet sein, wie Graphit oder leitenden Gläsern oder Kunststoffen oder dotierten Halbleitern, wie Silicium, Galliumarsenid, Germanium und dergleichen. Beispielsweise ist so ein monolithischer oder in Schichttechnik erzeugter Aufbau möglich.

## Patentansprüche

1. Meßwertaufnehmer zur polarographisch-amperometrischen Messung, insbesondere zur transcutanen Bestimmung des Sauerstoffpartialdrucks (tcpO₂) im Blut eines Lebewesens mittels einer polarographisch-amperometrischen Elektrodenanordnung nach CLARK, bei der an der Meßfläche (10) freiliegend und durch eine dünne, membranüberspannte Elektrolytschicht bedeckt wenigstens eine durch einen in ein Isoliermaterial (1a) eingebettetem Isolierdraht gebildete Arbeitselektrode (1), und wenigstens eine von einer galvanisch isolier enden Schicht (3a) umgebenen, in einem Körper aus wärmeleitendem Material, das eine Referenz (2) enthält, eingebetenen Gegenelektrode (3) eingesetzt sind, **dadurch gekennzeichnet,** daß die Gegenelektrode (3) durch einen das Isoliermaterial (1a) der Arbeitselektrode (1) wenigstens teilweise umschließenden Ring (3) aus inertem, leitendem oder dotiertem, halbleitendem Elektrodenmaterial gebildet ist.

2. Meßwertaufnehmer nach Anspruch 1, **dadurch gekennzeichnet**, daß die Arbeitselektrode die Kathode (1) und die Gegenelektrode die Anode (3) ist.

3. Meßwertaufnehmer nach Anspruch 1, **dadurch gekennzeichnet**, daß die Arbeitselektrode (1) als Microkathode durch einen dünnen, in ein Glasröhrchen eingeschmolzenen Edelmetalldraht ausgebildet ist.

4. Meßwertaufnehmer nach Anspruch 2, **dadurch gekennzeichnet**, daß der die Gegenelektrode bildende Ring aus Edelmetall besteht.

5. Meßwertaufnehmer nach Anspruch 3, **dadurch gekennzeichnet**, daß eine Mehrzahl von Microkathoden in etwa gleichem radialem Abstand vom Zentrum des Meßaufnehmers und in gleichem Winkelabstand gegeneinander in den Referenzkörper (2) eingesetzt ist und daß jede der Kathoden durch einen Anodenring (3) umgeben ist.

6. Meßwertaufnehmer nach Anspruch 3, **dadurch gekennzeichnet**, daß eine Mehrzahl von Kathodengruppen (1) in etwa gleichem radialem Abstand vom Zentrum des Meßwertaufnehmers eingesetzt ist und daß jede der Kathoden der Gruppen durch einen Anodenring (3) umgeben ist (Fig. 5).

7. Meßwertaufnehmer nach Anspruch 3 oder 5, **dadurch gekennzeichnet**, daß dieser als kombinierter Meßwertaufnehmer zur gleichzeitigen transcutanen Bestimmung des Kohlendioxidpartialdrucks (tcpCO₂) mit einer pH-Wert-Meßelektrode (5) ausgestattet ist.

8. Meßwertaufnehmer nach Anspruch 7, **dadurch gekennzeichnet**, daß die pH-Wert-Meßelektrode (5) in an sich bekannter Weise eine Iridium/Iridiumoxid-Elektrode ist.

9. Meßwertaufnehmer nach Anspruch 2, **dadurch gekennzeichnet**, daß der die Anode bildende Edelmetallring durch ein dünnes, das Isoliermaterial (1a) der Kathode (1) umschließendes Feinblechmaterial aus Edelmetall gebildet ist.

10. Meßwertaufnehmer nach Anspruch 2, **dadurch gekennzeichnet**, daß der die Anode bildende Edelmetallring durch eine auf das die Kathode (1) umschließende Isoliermaterial (1a) aufgedampfte, aufgesputterte oder eingebrannte oder mit Leitkleber aufgebrachte Edelmetallbeschichtung gebildet ist.

11. Meßwertaufnehmer nach Anspruch 2, **dadurch gekennzeichnet**, daß der die Anode bildende Edelmetallring aus der im wesentlichen gleichen Metallzusammensetzung besteht wie das die Kathode (1) bildende Material.

12. Meßwertaufnehmer nach einem der vorstehenden Ansprüche, **gekennzeichnet durch** eine direkte galvanische Verbindung zwischen der Referenz (2) und der Gegenelektrode (3).

13. Meßwertaufnehmer nach einem der vorstehenden Ansprüche, **dadurch gekennzeichnet**, daß in die Meßfläche eine Elektrolytspeicherrinne (4) eingearbeitet ist.

14. Meßwertaufnehmer nach Anspruch 13, **dadurch gekennzeichnet**, daß die Elektrolytrinne (4) durch eine Mehrzahl von in gleichem Winkelabstand gegeneinander angeordneten radialen Vertiefungen gebildet ist (Fig. 3).

15. Meßwertaufnehmer nach Anspruch 14, **dadurch gekennzeichnet**, daß die radial verlaufenden Vertiefungen durch eine ringförmige, umlaufende Rinne miteinander verbunden sind (Fig. 4).

16. Meßwertaufnehmer nach einem der vorstehenden Ansprüche 13 bis 15, **dadurch gekennzeichnet**, daß die Referenz (2) durch eine nur in der oder den Elektrolytrinnen (4) chlorierte Silberfläche gebildet ist.

17. Meßwertaufnehmer nach einem der vorstehenden Ansprüche**, dadurch gekennzeichnet**, daß die Referenz (2) durch wenigstens eine kleine Fläche, insbesondere aus Ag/AgCl, gebildet ist.

18. Meßwertaufnehmer nach einem der vorstehenden Ansprüche, **dadurch gekennzeichnet**, daß die die Gegenelektrode (3) bildenden Ringe in Schichttechnik bei planarem Aufbau durch eindotiertes Halbleitermaterial oder eindotierte Leitpasten gebildet ist.

## Claims

1. Measuring sensor for polarographic/amperometric measurement, in particular for the transcutaneous determination of the oxygen partial pressure (tcpO₂) in the blood of a living organism by means of a CLARK polarographic/amperometric electrode arrangement, in which, exposed on the measuring face (10) and covered by a thin electrolyte layer over which a membrane is stretched, at least one working electrode (1), formed by an insulating wire embedded in an insulating material (1a), and at least one counter electrode (3) which is surrounded by an electrically insulating layer (3a) and asked into [sic] in a body composed of heat-conducting material which contains a reference (2), are inserted, characterised in that the counter electrode (3) is formed by a ring (3) which at least partially encloses the insulating material (1a) of the working electrode (1) and is composed of inert, conducting or doped, semiconducting electrode material.

2. Measuring sensor according to Claim 1, characterised in that the working electrode is the cathode (1), and the counter electrode is the anode (3).

3. Measuring sensor according to Claim 1, characterised in that the working electrode (1) is formed as micro-cathode by a thin noble metal wire fused in a glass tube.

4. Measuring sensor according to Claim 2, characterised in that the ring forming the counter electrode is composed of noble metal.

5. Measuring sensor according to Claim 3, characterised in that a plurality of microcathodes is inserted, at approximately the same radial distance from the centre of the measuring sensor and at the same angular distance from one another, in the reference body (2), and in that each of the cathodes is encircled by an anode ring (3).

6. Measuring sensor according to Claim 3, characterised in that a plurality of cathode groups (1) is inserted, at approximately the same radial distance from the centre of the measuring sensor, and in that each of the cathodes of the groups is encircled by an anode ring (3) (Fig. 5).

7. Measuring sensor according to Claim 3 or 5, characterised in that the latter is, as combined measuring sensor for the simultaneous transcutaneous determination of the carbon dioxide partial pressure (tcpCO₂), equipped with a pH-measuring electrode (5).

8. Measuring sensor according to Claim 7, characterised in that the pH-measuring electrode (5) is an iridium/iridium oxide electrode in a manner known per se.

9. Measuring sensor according to Claim 2, characterised in that the noble metal ring forming the anode is formed by a thin sheet of noble metal material which encloses the insulating material (1a) of the cathode (1).

10. Measuring sensor according to Claim 2, characterised in that the noble metal ring forming the anode is formed by a noble metal coating which is applied to the insulating material (1a) enclosing the cathode (1) by deposition by evaporation or sputtering, or stoving or by use of conducting adhesives.

11. Measuring sensor according to Claim 2, characterised in that the noble metal ring forming the anode is composed of essentially the same metal composition as the material forming the cathode (1).

12. Measuring sensor according to one of the preceding claims, characterised by a direct electrical connection between the reference (2) and the counter electrode (3).

13. Measuring sensor according to one of the preceding claims, characterised in that an electrolyte storage channel (4) is incorporated in the measuring face.

14. Measuring sensor according to Claim 13, characterised in that the electrolyte channel (4) is formed by a plurality of radial recesses arranged at the same angular distance from one another (Fig. 3).

15. Measuring sensor according to Claim 14, characterised in that the radial recesses are connected together by an annular encircling channel (Fig. 4).

16. Measuring sensor according to one of the preceding Claims 13 to 15, characterised in that the reference (2) is formed by a silver surface which is chlorinated only in the electrolyte channel(s) (4).

17. Measuring sensor according to one of the preceding claims, characterised in that the reference (2) is formed by at least one small area, in particular composed of Ag/AgCl.

18. Measuring sensor according to one of the preceding claims, characterised in that the rings forming the counter electrode (3) is [sic] formed by coating techniques with a planar structure by use of doped semiconductor material or doped conducting pastes.

## Revendications

1. Transducteur pour la mesure polarographique-ampèremétrique, en particulier pour la détermination transcutanée de la pression partielle d'oxygène (tcpO₂) dans le sang d'un être vivant au moyen d'un arrangement d'électrodes polarographique-ampèremétrique selon CLARK, dans lequel sur la surface de mesure (10) sont montées libres et recouvertes par une mince couche d'électrolyte recouverte par une membrane, au moins une électrode de travail (1) constituée d'un fil isolé enrobé dans un matériau isolant (1a), et au moins une contre-électrode (3) entourée par une couche (3a) galvaniquement isolante, dans un corps en matériau thermiquement conducteur, qui contient une référence (2), caractérisé en ce que la contre-électrode (3) est formée par un anneau (3), entourant au moins partiellement le matériau isolant (1a) de l'électrode de travail (1), en un matériau d'électrode inerte conducteur ou dopé, semiconducteur.

2. Transducteur selon la revendication 1, caractérisé en ce que l'électrode de travail est la cathode 51) et la contre-électrode l'anode (3).

3. Transducteur selon la revendication 1, caractérisé en ce que l'électrode de travail (1) est réalisée sous la forme d'une microcathode par un fil de métal noble mince, fondu dans un capillaire de verre.

4. Transducteur selon la revendication 2, caractérisé en ce que l'anneau formant la contre-électrode est en métal noble.

5. Transducteur selon la revendication 3, caractérisé en ce qu'une pluralité de microcathodes est disposée à écartement radial sensiblement identique du centre du transducteur et à écartement angulaire égal les unes par rapport aux autres dans le corps de référence (2), et en ce que chacune des cathodes est entourée par un anneau d'anode (3).

6. Transducteur selon la revendication 3, caractérisé en ce qu'une pluralité de groupes de cathodes (1) est disposée à écartement radial sensiblement identique du centre du transducteur, et en ce que chacune des cathodes des groupes est entourée par un anneau d'anode (3) (Figure 5).

7. Transducteur selon la revendication 3 ou 5, caractérisé en ce que celui-ci, en tant que transducteur combiné pour la détermination transcutanée simultanée de la pression partielle de gaz carbonique (tcpCO₂), est équipé d'une électrode de mesure de valeur de pH (5).

8. Transducteur selon la revendication 7, caractérisé en ce que l'électrode de mesure de la valeur de pH (5) est, d'une manière en soi connue, une électrode en iridium/oxyde d'iridium.

9. Transducteur selon la revendication 2, caractérisé en ce que l'anneau de métal noble formant l'anode est formé par une tôle mince de métal noble entourant le matériau isolant (1a) de la cathode (1).

10. Transducteur selon la revendication 2, caractérisé en ce que l'anneau de métal noble formant l'anode est formé par une couche de métal noble déposée par vaporisation, pulvérisation ou cuisson ou à l'aide d'une colle conductrice sur le matériau isolant (1a) entourant la cathode (1).

11. Transducteur selon la revendication 2, caractérisé en ce que l'anneau de métal noble formant l'anode est sensiblement de la même composition métallique que le matériau formant la cathode (1).

12. Transducteur selon l'une quelconque des revendications précédentes, caractérisé par une liaison galvanique directe entre la référence (2) et la contre-électrode (3).

13. Transducteur selon l'une quelconque des revendications précédentes, caractérisé en ce que dans la surface de mesure est usinée une rigole réservoir d'électrolyte (4).

14. Transducteur selon la revendication 13, caractérisé en ce que la rigole d'électrolyte (4) est formée par une pluralité de cavités radiales disposés entre elles à écartement angulaire uniforme (Figure 3).

15. Transducteur selon la revendication 14, caractérisé en ce que les cavités s'étendant radialement sont réunies entre elles par une rigole circulaire annulaire (Figure 4).

16. Transducteur selon l'une quelconque des revendications précédentes 13 à 15, caractérisé en ce que la référence (2) est formée par une surface de chlorure d'argent uniquement dans la ou les rigoles d'électrolyte (4).

17. Transducteur selon l'une quelconque des revendications précédentes, caractérisé en ce que la référence (2) est formée par au moins une petite surface, en particulier en Ag/AgCl.

18. Transducteur selon l'une quelconque des revendications précédentes, caractérisé en ce que les anneaux formant la contre-électrode (3) sont réalisés par stratification en structure planaire par un matériau semiconducteur dopé ou des pâtes conductrices dopées.
